# EUROPÄISCHE PATENTSCHRIFT

(11) **EP 1 235 571 B1**
(45) Veröffentlichungstag und Bekanntmachung des Hinweises auf die Patenterteilung: **08.09.2004**
(21) Anmeldenummer: 00993238.5
(22) Anmeldetag: 29.11.2000
(51) Int. Cl.: A61K 31/4045, A61P 5/26, A61P 17/14

(54) **VERWENDUNG VON MELATONIN ZUR BEHANDLUNG DER ANDROGENETISCHEN UND DIFFUSEN ALOPEZIE VOM WEIBLICHEN TYP**
USE OF MELATONIN FOR TREATING ANDROGENETIC AND DIFFUSE FEMALE TYPE ALOPECIA
UTILISATION DE MELATONINE POUR TRAITER L'ALOPECIE ANDROGENETIQUE ET DIFFUSE DU TYPE FEMELLE

(30) Priorität: 30.11.1999 DE 19957710; 18.02.2000 US 506442
(43) Veröffentlichungstag der Anmeldung: 04.09.2002
(73) Patentinhaber: Asat AG Applied Science & Technology, 6302 Zug (CH)
(72) Erfinder: ELSNER, Peter, CH-8706 Meilen (CH)
(74) Vertreter: Weiss, Wolfgang, Dipl.-Chem. Dr.
(86) Internationale Anmeldenummer: PCT/EP2000/011976
(87) Internationale Veröffentlichungsnummer: WO 2001/039771

(56) Entgegenhaltungen:
- WO-A-98/05298
- WO-A-99/32072
- ES-A- 2 101 660

## Beschreibung

Die Erfindung betrifft die Verwendung von Melatonin und Melatonin enthaltenden Präparaten zur Behandlung der androgenetischen Alopezie vom weiblichen Typ.

Melatonin (N-Acetyl-5-Methoxytryptamin) ist ein unter dem Einfluß β-adrenerger Rezeptoren mit zirkadianem Rhythmus von der Glandula pinealis produziertes und sezerniertes Hormon mit breitem Wirkungsspektrum [1,2]. Zwar sind die Mechanismen der Melatonin-Wirkung im einzelnen noch nicht umfassend geklärt, jedoch scheint Melatonin die Adaption des Organismus an Umweltreize, insbesondere Licht und Temperatur, zu steuern. Die Toxizität von Melatonin ist auch bei oraler Gabe von mehreren Gramm pro Tag nicht nachweisbar [3]. Bei der topischen Anwendung in einer Nanokolloidzubereitung sowie von alkoholischen Lösungen zeigte es bei über 60 Probanden keine lokalen oder systemischen Nebenwirkungen. Tierexperimentelle Studien weisen darauf hin, dass die systemische Gabe von Melatonin die Dichte und Struktur von Fellhaaren verbessert [4,5].

Der Einfluß der Dauer der Tageslichtexposition auf den saisonalen Fellwechsel ist für das Schaf [6], die Kaschmirziege und einige andere Ziegenarten [7], Rotwild [8] und Nerz [9] beschrieben worden. Die mitotische Aktivität der Sekundärfollikel und das daraus resultierende Haarwachstum steigt mit Beginn des Sommers bis zum Winter an, um im Frühjahr zu sistieren. Nach einer Ruhephase, in der die Haare der Primärund Sekundärfollikel ausfallen, beginnt mit Induktion einer neuen Anagenphase ein neuer Wachstumszyklus.

Dieser Zyklus des Haarwachstums und der Mauserung ist gestört, wenn die Zirbeldrüse entfernt wird [5]. Welch [10] konnte zeigen, dass durch Melatoningabe bei Kaschmirziegen die Initialisierung der Wachstumsaktivität der Sekundärfollikel im Frühjahr beschleunigt werden kann. In Neuseeland wurden Ziegen über 14 Tage mit Melatonin behandelt, um das Frühjahrswachstum einzuleiten, und mit unbehandelten Ziegen verglichen. Die histologische Untersuchung von Biopsien der Haut, die während der 14 Tage entnommen wurden, zeigte bei den Ziegen, die mit Melatonin behandelt worden waren, eine Induktion des Haarwachstums durch Übertritt des Haarfollikels aus der Telogenphase in die Proanagenphase, während die Haarfollikel der unbehandelten Ziegen in der Telogenphase verblieben [11].

Bei in vitro-Untersuchungen von Haarfollikeln der Kaschmirziege im Haarorgankulturmodell konnte ebenfalls der Einfluß von Melatonin auf das Haarwachstum des Follikels nachgewiesen werden. Die Gabe von Melatonin in Konzentrationen ab 150 mg/ml führte im 24 Stunden-Intervall zu einem stärkeren Wachstum der Haarschäfte als die niedrigere Dosierung und die Kontrolle. Über die Gesamtdauer von 120 Stunden waren die Unterschiede im Längenwachstum signifikant (p=0,05). Die besten Wachstumsraten und maximales Gesamtwachstum nach 120 Stunden wurden bei der Anwendung der Melatoninkonzentration von 300 ng/l verzeichnet [12].

Das US-Patent 4,476,674 offenbart die Verwendung von Melatonin zur Behandlung verschiedener Erkrankungen des Menschen, die mit Haarausfall einhergehen, nämlich der Behandlung von toxischer, z.B. durch Behandlung mit Arzneimitteln induzierter Alopezie und der Behandlung der Alopezie vom männlichen Typ. Ein Hinweis auf die Anwendung von Melatonin zur Behandlung bei der androgenetischen Alopezie vom weiblichen Typ findet sich nicht.

Die androgenetische Alopezie vom weiblichen Typ unterscheidet sich signifikant von einer toxischen Alopezie. Wesentliche Unterschiede bestehen auch zur androgenetischen Alopezie vom männlichen Typ und zwar hinsichtlich der klinischen Ausprägung, des Wirkmechanismus und der Therapierbarkeit.

### 1. Unterschiede in der klinischen Ausprägung

Obwohl die androgenetische Alopezie bei beiden Geschlechtern vorkommt, ist die klinische Ausprägung typischerweise unterschiedlich.

Das Haarausfallsmuster vom männlichen Typ (male pattern alopecia) wurde bereits 1942 von Hamilton beschrieben und von Eblin und Rook 1972 modifiziert. Dabei zeigt sich in Stadium 1 eine ausgeprägte Geheimratseckenbildung, die ab Stadium 2 mit zunehmender Lichtung des okzipitalen Kopfhaares einhergeht und dann mit zunehmendem Konfluens beider Areale im Stadium 4 das Bild einer weitgehend kahlen Kopfhaut mit einem Resthaarkranz im okzipitalen Bereich ergibt [13, 14].

Das Haarausfallsmuster vom weiblichen Typ (female pattern alopecia) unterscheidet sich deutlich vom männlichen Typ, da bei dieser Form keine Geheimratsecken auftreten und nie eine völlige Kahlheit der Kopfhaut resultiert. Diese Form wurde von Ludwig in drei Stadien eingeteilt, wobei sich mit zunehmendem Stadium vor allem der zentroparietale (Scheitel-) Bereich diffus lichtet. Auch bei fortgeschrittener Alopezie der Frau steht meist noch ein dünner Haarkranz von 1 bis 2 cm zwischen Stirn und gelichteter Scheitelregion [15].

### 2. Unterschiede im Wirkmechanismus

Unter der Bezeichnung "androgenetische Alopezie" werden Haarausfallformen verstanden, die beim Vorliegen einer genetischen Disposition unter dem Einfluß von Androgenen entstehen. Sie sind mit einer Beschleunigung des Haarzyklus verbunden, die zur Vermehrung von Telogenhaaren und zunehmendem Auftreten von dünnen Miniaturhaaren führt.

Beim Mann führen die hohen Androgenspiegel bei gegebener anlagebedingter Sensibilität der Haarwurzeln auf Androgene regelmäßig zur männlichen Glatzenbildung.

Bei der Frau, die 10mal niedrigere Androgenspiegel als der Mann aufweist, ist weniger die absolute Androgenmenge als die erhöhte Endorganempfindlichkeit auf Androgene für die Entwicklung des Haarausfalls verantwortlich. Dieser Mechanismus führt bei über 90% der Frauen zur female pattern alopecia, während in den übrigen Fällen auch eine male pattern alopecia auftreten kann. Hierbei finden sich oft erhöhte Androgenspiegel [16]. Ebenso kann beim Mann eine seltene female pattern alopecia auftreten, die auch in diesem Fall keine Ähnlichkeit mit der male pattern alopecia hat und auch nicht in diese übergeht.

### 3. Unterschiede in der Therapierbarkeit

Es zeigen sich auch Unterschiede in den therapeutischen Wirkmechanismen.

Die in den letzten Jahren umfangreich durchgeführten Studien zur Alopezie des Mannes zeigten ein sehr gutes Ansprechen eines 5-alpha-Reduktasehemmers (Finasterid), der die Umwandlung von Testosteron zu Dihydrotestosteron hemmt. In 80% der Fälle konnte ein Stoppen des Haarausfalls und in 66% der Fälle eine geringe bis deutliche Besserung der Haardichte erzielt werden [17]. Somit scheint der Pathomechanismus des männlichen Haarausfalltyps doch eher durch die umgesetzte Menge von Androgenen als durch die Empfindlichkeit der Wurzeln bedingt zu sein.

Bislang nicht veröffentlichte Daten zu Versuchen mit Finasterid zeigten bei postmenopausalen Frauen keine überlegene Wirksamkeit gegenüber Placebo. Dies gibt den entscheidenden Hinweis auf die erhöhte Sensibilität der Haarwurzel auf die bei der Frau vorliegenden geringen Mengen von Androgenen.

Darüber hinaus ist die Anwendung von Finasterid bei Frauen im gebährfähigen Alter kontraindiziert, da erniedrigte Spiegel von Dihydrotestosteron, dem aktiven Metaboliten von Testosteron, bei einem männlichen Feten zu Fehlbildungen des äußeren Genitals führen können.

In einer zur vorliegenden Patentanmeldung führenden wissenschaftlichen Untersuchung wurde nun festgestellt, dass Melatonin ein geeigneter Wirkstoff zur Behandlung der androgenetischen Alopezie vom weiblichen Typ ist. Es wurden signifikant bessere Ergebnisse der Melatoninanwendung bei der androgenetischen Alopezie der Frau verglichen mit denen der diffusen Alopezie der Frau in bezug auf die Telogenraten gefunden. Die Anwendung von Melatonin bei der androgenetischen und diffusen Alopezie der Frau ist somit als spezielle medizinische Indikation von der androgenetischen Alopezie des Mannes abzugrenzen und impliziert ein neuartiges Wirkprinzip. Es kann vermutet werden, dass Melatonin beim androgenvermittelten Mechanismus der Frau modulierende Eigenschaften entwickelt, die beim Mann aufgrund der hohen Androgenkonzentration nicht greifen.

Zur Behandlung der androgenetischen Alopezie vom weiblichen Typ wird vorzugsweise ein Melatonin-Präparat topisch appliziert. Die Applikation kann dabei in Form von Sprays, Lösungen, Lotionen, Cremes oder Salben erfolgen. Die Präparate können neben dem Wirkstoff geeignete Träger-, Verdünnungs- oder Hilfsstoffe sowie gegebenenfalls weitere pharmakologisch aktive Wirkstoffe wie etwa Vitamine, z.B. die Vitamine C, E oder/und H enthalten. Beispiele für geeignete Träger sind Wasser, wässrige Puffer, Alkohol oder lipophile Substanzen. Gegebenenfalls können Hilfsstoffe eingesetzt werden, welche die Absorption und Penetration des Wirkstoffs verbessern (siehe US-Patent 4,746,674).

Das Melatonin-Präparat kann beispielsweise eine Wirkstoffkonzentration von 0,001 bis 1% (Gew./Gew.) aufweisen. Bevorzugt liegt die Wirkstoffkon-zentration in Bereichen von 0,01 bis 0,5% (Gew./Gew.). Die täglich appli-zierte Wirkstoffdosis hängt von der Schwere der Erkrankung und der Art der Applikation ab. So haben sich Wirkstoffdosierungen von 0,001 bis 10 mg, vorzugsweise von 0,01 bis 10 mg und besonders bevorzugt von 0,1 bis 5 mg pro Tag und Patientin als geeignet erwiesen.

Durch die Verabreichung von Melatonin wird überraschenderweise eine Reduktion der Telogenrate erzielt. Die Telogenrate kennzeichnet in dem Bereich von unter 20% einen physiologischen Haarausfall, d.h. dass ausfallende Haare durch nachwachsende Haare ersetzt werden. Eine Telogenrate von 20% ist dabei als Obergrenze eines Normbereichs zu betrachten, wobei sich die Mehrzahl der Befunde in einem Bereich von 5% bis 15% bewegt. Übersteigt die Telogenrate 20%, kommt es zu einem Nettoverlust des Kopfhaares, bei dem ausfallende Haare nicht mehr vollständig durch nachwachsende Haare ersetzt werden können. Daraus resultiert eine zunehmende Ausdünnung des Kopfhaares. Durch Verabreichung von Melatonin konnte eine Reduktion der Telogenrate von einem über die Norm erhöhten Wert in den Normbereich, d.h. unter 20% erzielt werden.

Weiterhin wird bei Patientinnen mit androgenetischen Alopezie des weiblichen Typs durch die Melatoningabe eine Vergrößerung des Haardurchmessers oder/und eine Erhöhung der Widerstandskraft der Haare, insbesondere bei frontalen Haaren, aber auch bei okzipitalen Haaren gefunden.

Auch bei der diffusen Alopezie des weiblichen Typs konnte eine Wirkung des Melatonin-Präparats gefunden werden. Diese Wirkung war zwar nicht so ausgeprägt wie bei der androgenetischen Alopezie, dennoch wurde insbesondere bei Okzipitalhaaren eine signifikante Vergrößerung des Haadurchmessers und eine Steigerung der Dehnungsfähgigkeit beobachtet.

Außerdem konnte eine statistisch signifikante Wirkung (p=0,02) der Melatoninbehandlung, insbesondere hinsichtlich der Anzahl von Anagen-Haarfollikeln, nachgewiesen werden.

Die Erfindung betrifft somit auch die Verwendung von Melatonin zur Vergrößerung des Haardurchmessers oder/und Erhöhung der Dehnungsfähigkeit oder/und zur Erhöhung der Anzahl von Anagen-Haarfollikeln bei diffuser Alopezie, insbesondere bei okzipitalen Haaren und weiblichen Patientinnen.

Die Melatoninplasmaspiegel stiegen unter der Melatoningabe verglichen mit einer Placebobehandlung zwar an, nicht jedoch über den physiologischen Nachtspiegel von 250 pg/ml. Bei dem mit Melatonin behandelten Testpersonen zeigte sich gegenüber der Plazebogruppe kein gehäuftes Auftreten von Müdigkeit, so dass das Sicherheitsprofil der verwendeten Melatoninapplikation als gut einzustufen war.

Weiterhin soll die Erfindung im Rahmen der folgenden Beispiele erläutert werden.

### Beispiele

### 1. Methodik

### Studiendesign

Die Prüfung wurde als randomisierte, placebokontrollierte Doppelblind-Studie an 40 Patientinnen mit einer diffusen oder einer androgenetischen, nicht vernarbenden Alopezie, die sich nach Aufklärung zur Teilnahme an der Untersuchung bereiterklärten, durchgeführt.

### Prüfverfahren

Es erfolgte eine tägliche abendliche topische Behandlung der Kopfhaut mit 8 Hüben Melatonin-Spray 0,1 % ä 0,128 ml entsprechend 1,024 ml täglich oder Gabe von melatoninfreier alkoholischer Lösung (Placebo). Die Behandlungsdauer betrug 6 Monate. Die Bestimmung des Haarwachstums und der mechanischen Haarqualität erfolgte mittelt Trichogrammen sowie Messungen des Haarschaftdurchmessers und der Zugfestigkeit. Es wurden Kontrollen des Plasma-Melatoninspiegets durchgeführt.

### Testpersonen

Es wurden 40 haut- und allgemeingesunde Frauen mit androgenetischer oder diffuser Alopezie im Alter von 20 bis 70 Jahren getestet. Es wurde darauf geachtet, dass keine Schwangerschaft oder Stillzeit, keine dermatologische Lokaltherapie auf der Kopfhaut und keine vernarbende Alopezie vorlagen. Weiterhin wurden keine haarkosmetischen Maßnahmen während der Studie vorgenommen.

### Auswaschphase

Jede Patientin mußte vor Studienbeginn eine einwöchige Auswaschphase mit einem neutralen Shampoo (Every-Day-Shampoo®, Fa. Sebapharma, Boppard, Germany) durchführen.

### Testsubstanz

Es wurden Melatonin 0, 1 % in alkoholischer Lösung (30% Ethanol, 70% Wasser, v/v) in lichtgeschützten Aluminium-Fläschchen mit standardisiertem Sprühkopf sowie Placebo in alkoholischer Lösung (30% Ethanol, 70% Wasser; v/v) in lichtgeschützten Aluminium-Fläschchen mit standardisiertem Sprühkopf jeweils in nummernkodierter randomisierter Verteilung verwendet.

Die Testprodukte wurden zu Beginn der Studie als ein Paket mit 6 Sprühfläschchen für die Behandlungszeit von 6 Monaten ausgehändigt. Ein Fläschchen enthielt jeweit 33 ml entsprechend einer Monatsmenge.

### Zielparameter

### Trichogramm:

Zur Untersuchung der Anagen- und Telogenraten wurden Trichogramme angefertigt. Dabei wurden jeweils 5 Tage nach der letzten Haarwäsche in standardisierter Weise 30 bis 50 Haare frontal etwa 2 cm von der Stirn-Haar-Grenze und seitlich von der Sagittallinie durch Epilation mit einer speziellen Haarklemme gewonnen. Eine 2. Epilation wurde okzipital 2 cm seitlich der Protuberantia occipitalis entnommen. Die Trichogramme wurden vor Beginn der Studie, und nach 3 und 6 Monaten Behandlung durchgeführt. Die gewonnenen Haarwurzeln wurden auf Objektträger aufgebracht, mittels Lichtmikroskop bei 20-facher Vergrößerung analog ausgezählt und die prozentualen Verhältnisse der Anagen-, Telogen- und dystrophen Haare errechnet.

### Haarschaftmessungen:

Aus den für das Trichogramm gewonnenen Haarproben wurden frontal und okzipital jeweils mindestens 10 Haare für die Haarschaftmessungen verwendet. Diese Proben wurden vor Beginn der Präparat-Applikation und nach 3 und 6 Monaten Behandlung untersucht.

### Durchmesser

Zur Messung des Durchmessers und der haarmechanischen Eigenschaften wurden jeweils 5 Haare aus dem frontalen und okzipitalen Entnahmebereich der Trichogramme verwendet.

### Zerreißkraft

Zur Messung der Kraft, die aufgewendet werden mußte, um das Haar zum Zerreißen zu bringen, wurden die Haare mittels einer computergesteuerten Meß- und Regeleinheit mit konstantgesteigerter Kraft (Newton) bis zum Zerreißpunkt gedehnt.

### Zerreißstrecke

In analoger Weise wie die Zerreißkraft ermittelt wurde, konnte durch die Meßeinheit die Wegstrecke der Dehnung bis zum Zerreißpunkt ermittelt werden. Die Dehnungsstrecke wurde in mm gemessen.

### Melatonin-Plasmaspiegel:

Melatonin-Plasmaspiegel wurden mittels eines kommerziell erhältlichen Radioimmuntest (RIA, DPC Biermann GmbH, Bad Neuheim, Deutschland) bestimmt.

### Patientenfragebögen

Unerwünschte Arzneimittelnebenwirkungen wurden über Patientenfragebögen ermittelt. Am Ende der Studie wurde ein zusätzlicher Abschlußfragebogen von den Patientinnen ausgefüllt, in welchem sie sich zum Erfolg der Therapie, zum zeitlichen Auftreten von Nebenwirkungen und zur Darreichungsform des Präparates äußern konnten.

### Daten und Statistik

Die gesamten Einzeldaten aus den Trichogrammuntersuchungen, den Haarschaftmessungen und den Melatoninmessungen wurden in Microsoft Excel erfaßt und Mittelwerte und Standardabweichungen errechnet. Die statistische Analyse der Meßwerte zu den verschiedenen Zeitpunkten in den beiden Behandlungsgruppen wurde durch das SPSS Statistikprogramm durchgeführt.

### 2. Ergebnisse

### Studienpopulation

Es wurden insgesamt 40 weibliche Patientinnen in die Studie aufgenommen, wobei 12 Frauen eine androgenetische und 28 Frauen eine diffuse Alopezie aufwiesen. Die klinische Schwere der androgenetischen Alopezie reichte vom Stadium Ludwig I bis III, die der diffusen Alopezie von gering bis mittelgradig ausgeprägt. Es bestand bei keiner Patientin eine internistische Erkrankung oder eine andere lokale Erkrankung der Kopfhaut außer der Alopezie.

### Trichogramme

Die Trichogramme wurden nach Plan am Beginn des 6-monatigen Untersuchungszeitraumes, nach dem 3. Monat und dem 6. Monat (Endmessung) durchgeführt. Für die Gesamtauswertung wurden die Anagen- und Telogenraten beider Erkrankungsgruppen zusammengefaßt.

### Frontale Trichogramme

Die Auswertung der frontalen Trichogramme zeigte in der Placebo-Gruppe eine relativ gleichbleibende Anagenrate von 82,2% vor der Therapie, 82% nach zwei Monaten und 82,2% nach sechs Monaten. Die Telogenrate verhielt sich dementsprechen ähnlich, wobei eine geringgradige Abnahme von 17,2% auf 16,8% am Ende des Untersuchungszeitraumes zu verzeichnen war. Die Rate der dystrophen Haare bewegte sich im normalen Bereich von unter 4%. In der Verum-Gruppe konnte eine geringe Zunahme der Anagenrate von 80,4% auf 82,6% beobachtet werden. Die Telogenrate nahm von 18,9% auf 16,8% und 15,9% ab. Die dystrophen Haare wiesen mit einem Anteil von 0,6% bis 2,2% eine normale Rate auf. Die Unterschiede der Anagen- bzw. Telogenraten nach 3 bzw. 6 Monaten Behandlung gegenüber den Ausgangswerten waren sowohl in der Placeboals auch in der Verum-Gruppe nicht signifikant.

### Okzipitale Trichogramme

Bei Betrachtung der okzipitalen Trichogramme zeigte sich in der Placebo-Gruppe zunächst eine Abnahme der Anagenrate von 80,4% auf 79,5% bis zum Monat 3 und eine anschließende Zunahme auf 84,1 % zum Monat 6.

Die Telogenrate nahm bis zum Monat 3 von 16,8% auf 19,2% zu und zum Monat 6 auf 14,0% ab.

Die Veränderungen in der Placebo-Gruppe waren weder für die Anagennoch für die Telogenraten signifikant.

Die Anagenraten der Verum-Gruppe zeigten hingegen eine deutliche kontinuierliche Zunahme von 76,3% auf 78,8% (Monat 3) und 85% zum Monat 6. Der Unterschied der Anagenrate zwischen Ausgangsbefund und Monat 6 war signifikant (p ≤ 0,021).

Noch ausgeprägtere Verändungen sowohl nach 3-monatiger als auch nach 6-monatiger Behandlung zeigten sich in der Telogenrate der Verum-Gruppe. Sie fiel von Monat 1 zu Monat 3 von 23,03% auf 18,1% ab. Bis zum Monat 6 konnte eine Abnahme auf 12,5% erzielt werden. Damit wurde durch die 6-monatige Behandlung mit Verum eine Reduktion der Telogenrate auf etwa die Hälfte erzielt. Der Unterschied von Monat 3 zu Monat 6 war mit p≤0,048 signifikant und über den gesamten Beobachtungszeitraum von 6 Monaten war er hochsignifikant (p≤0,005).

### Analyse der Diagnosegruppen (Alopecia androgenetica vs. Alopecia diffusa)

Aufgrund der signifikanten Unterschiede in den okzipitalen Trichogrammen der Verum-Gruppe wurde eine Einteilung in Abhängigkeit der verschiedenen Diagnosen vorgenommen.

### Androgenetische Alopezie

Die 12 Patientinnen mit androgenetischer Alopezie zeigten unter Placebo (n = 6) gleichbleibende Anagen- und Telogenraten von 78,22% bis 82,11 % bzw. 17,6% und 17,2%. Die Anagenraten bei Patientinnen der Verum-Gruppe (n = 6) nahmen von 73,6% auf 87,8% zu. Bei der Telogenrate konnten noch deutlichere Unterschiede mit einer Abnahme von 26,5% auf 18,3% (Monat 3) und 10,8% (Monat 6) festgestellt werden. Dabei war die Abnahme von Ausgangsbefund zu Monat 6 mit p ≤ 0,035 signifikant.

### Diffuse Alopezie

Bei den 28 Patientinnen mit diffuser Alopezie zeigte sich unter Placebogabe (n = 14) eine Zunahme der Anagenrate von 81,39% auf 84,89% und eine Abnahme der Telogenrate von 16,4% auf 12,66%.

Bei Patientinnen der Verum-Gruppe (n=14) konnte eine Zunahme der Anagenrate von 77,4% auf 83,7% verzeichnet werden. Die Telogenrate nahm von 21,6% auf 13,2% ab. Ein Vergleich der Melatonin- und Placebogruppe zeigte statistisch signifikante Unterschiede bei Patientinnen mit diffuser Alopezie (p = 0,02) hinsichtlich der Zunahme der Anagenrate.

### Messung der physikalischen Haarparameter

Messungen des Haardurchmessers von frontal entnommenen Haaren ergaben eine Vergrößerung des Durchmessers in 67% der Patienten der Verumgruppe mit der Diagnose Androgenetische Alopezie. Die Widerstandskraft von Haaren von Patientinnen unter Melatoninbehandlung nahm von 0,56 N auf 0,61 N nach 6 Monaten Behandlung zu. In den Frontalhaaren zeigten sich sonst keine weiteren Verbesserungen gegenüber der Placebogruppe bezüglich der physikalischen Haarparameter.

In Untersuchungen an okzipital entnommenen Haaren zeigte sich in 55% von Patientinnen unter Melatoninbehandlung eine Vergrößerung des Haardurchmessers und in 70% der Patientinnen eine Steigerung der Dehnungskraft. Die Verbesserungen durch Melatonin wurden besonders in der Gruppe mit diffuser Alopezie beobachtet (86%).

### Melatonin-Plasmaspiegel

Die Melatonin-Plasmaspiegel lagen in der Placebo-Gruppe bei Werten zwischen 2 und 10 pg/ml mit einzelnen Spitzenwerten von bis zu 80 pg/ml. Es konnten teilweise erhebliche intra- und interindividuelle Unterschiede festgestellt werden.

In der Verum-Gruppe lagen die Melatonin-Plasmaspiegel zum Meßzeitpunkt 1 vor Applikation im Bereich von 2 bis 10 pg/ml und stiegen zu den folgenden Meßzeitpunkten auf Werte von durchschnittlich 30 bis 50 pg/ml an. Einzelne Spitzenwerte lagen bei 180 pg/ml. Es zeigten sich auch in dieser Gruppe erhebliche intra- und interindividuelle Unterschiede der Meßwerte. Trotz der Applikation von Melatonin wurde in dieser Gruppe der physiologische durchschnittliche Melatonin-Peak in der Nacht von 250 pg/ml nicht überschritten.

Weiterhin kam es nicht zu einer Kumulation des Melatonin-Serumspiegels. Die Melatonin Plasmaspiegel blieben konstant zwischen 30 und 50 pg/ml über den Untersuchungszeitraum von 6 Monaten.

### Patientenfragebögen

### Erfolg der Therapie

Der Haarausfall wurde in der Placebo-Gruppe von insgesamt 56% der Patientinnen als gebessert, von 36% als gestoppt und von 13% als verschlechtert eingestuft.

In der Verum-Gruppe gaben 69% der Patientinnen an, dass der Haarausfall sich verbessert hätte, bei 23% sei der Haarausfall gestoppt worden und nur bei 8% hatte sich der Haarausfall verschlechtert.

### Akzeptanz des Präparats

Die Präparatgröße wurde von der Mehrzahl der Anwenderinnen (85%) als gut eingestuft. Die Sprayform wurde von 91 % als geeignet angegeben und die Anwendung allgemein von nahezu allen Patientinnen (97%) als praktisch eingestuft.

### 3. Diskussion

In der vorliegenden Studie konnte eine signifikante Wirksamkeit des topischen 0,1%-igen Melatoninpräparates in der Dosierung von 1 ml täglich auf die Telogenrate im Okzipitalbereich gezeigt werden. Während die Qualität der Haare hinsichtlich ihres Durchmessers und ihrer Dehnungseigenschaften durch das Melatonin-Präparat nicht signifikant im Unterschied zum Placebo beeinflußt werden konnte, zeigten sich in den Trichogrammbefunden klare Unterschiede zwischen Verum und Placebo. Bei der Gesamtauswertung der Befunde aller Patientinnen zeigte sich vor allem bei den okzipitalen Trichogrammen am Ende des 6-monatigen Behandlungszeitraumes eine signifikante Zunahme der Anagenhaare (p ≤ 0,021) und eine Abnahme der Telogenhaare (p ≤ 0,005). Bei Betrachtung der Telogenhaare zeigte sich sogar ein signifikanter Unterschied von Monat 3 zu Monat 6 (p ≤ 0,48). Die Rate der dystrophen Haare lag stets im Normbereich, so dass keine schädigenden Wirkungen des Präparates auf das physiologische Haarwachstum beobachtet werden konnten. Da die Bestimmung des Haarwurzelstatus durch das Trichogramm ein prozentuales Verhältnis von Anagen-, Telogen- und dystrophen Haaren darstellt, ergibt sich zwangsläufig, dass Veränderungen eines Haartyps immer auch Veränderungen des anderen Typs bedingen. Trotzdem ist die Bestimmung der Telogenrate als Parameter des Haarausfalls als besonders bedeutsam einzustufen, da die lichtmikroskopische Beurteilung des Telogenhaares als pigmentloses, kolbenförmiges Haar ohne Wurzefscheide eindeutig vorzunehmen ist. Unter der Klasse der Anagenhaare hingegen werden verschiedene Haartypen subsummiert wie z.B. Anagenhaare mit und ohne Wurzelscheiden, abgebrochene Haare und dysplastische Anagenhaare. Hier ergeben sich teilweise Diskriminierungsschwierigkeiten mit den dystrophen Haaren, die nicht den Anagenhaaren zugerechnet werden. Aus diesem Grund wurden alle Trichogramme, die einen unschlüssigen Befund hinsichtlich der dystrophen Haare und Anagenhaare aufwiesen, einer Plausibilitätskontrolle und erneuten kompletten Auszählung unterzogen.

Die Telogenrate kennzeichnet in dem Bereich von unter 20% einen physiologischen Haarausfall, d.h. dass die ausfallenden Haare durch nachwachsende Haare ersetzt werden. Die Rate von 20% ist als Obergrenze eines Normbereiches zu betrachten, bei dem die Mehrzahl der Befunde sich in einem Bereich von 5% bis 15% bewegen. Übersteigt die Telogenrate 20%, kommt es zu einem Nettoverlust des Kopfhaares, bei dem die ausfallenden Haare nicht mehr vollständig durch nachwachsende Haare ersetzt werden können. Daraus resultiert eine zunehmende Ausdünnung des Kopfhaares. In der vorliegenden Studie konnte die signifikante Reduktion der Telogenrate im okzipitalen Bereich festgestellt werden, wo es zu einer Abnahme von 23% auf 12,5% kam. Die zum Beginn der Studie leicht über die Norm erhöhte Telogenrate lag somit am Ende des 6-monatigen Behandlungszeitraumes völlig im Normbereich. Bei der Auswertung getrennt nach Diagnosegruppen zeigte sich, dass die Gruppe mit androgenetischer Alopezie am meisten von der Anwendung des Präparates insbesondere im Bereich der okzipitalen Kopfhaut profitierte. Das Melatonin-Präparat erzielte bei einer Ausgangsrate von 26,5% Telogenhaaren eine Reduktion auf 18,3% nach 3 Monaten Anwendung und auf 10,8% nach 6 Monaten. Die Unterschiede waren von Monat 1 zu Monat 6 mit p ≤ 0,035 signifikant.

Die positiven Wirkungen auf die Anagenraten der frontalen Haarproben waren ebenfalls durch die Anwendung des Melatonin-Präparates stärker als die des Placebos und zeigten statistisch signifikante Unterschiede, insbesondere bei Patientinnen mit diffuser Alpezie.

Bei der diffusen Alopezie ist die Wirkung des Melatoninpräparates zwar vorhanden, jedoch nicht derart ausgeprägt wie bei der androgenetischen. Die diffuse Alopezie stellt eine nicht näher spezifizierte symptomorientierte Diagnose dar, bei der verschiedene Ursachen wie nutritive Faktoren, Noxen und Perfusionsstörungen der Kopfhaut das physiologische Gleichgewicht des Haarwachstums stören. Hier kann Melatonin als Förderer der Anageninduktion ebenfalls wirksam sein, jedoch nur unter der Voraussetzung, dass andere schädigende Faktoren nicht überwiegen.

Die Melatonin-Plasmaspiegel stiegen unter der Melatoningabe verglichen mit der Placebobehandlung zwar an, jedoch nicht über die physiologischen Nachtspiegel von 250 pg/ml. Die zu erwartende Wirkung von erhöhten Melatonin-Plasmaspiegeln auf die Vigilanz der Testpersonen ist durch die Patientenfragebögen erfaßt worden. Es zeigte sich gegenüber der Placebogruppe kein gehäuftes Auftreten von Müdigkeit, so dass das Sicherheitsprofil der verwendeten Melatoninpräparation bei topischer Applikation auf der Kopfhaut als gut einzustufen ist.

### Literaturstellen

1. Arendt J. The pineal. In: Touitou Y, Haus E, eds. Biological Rhythm in Clinical and laboratory medicine, 1992
2. Cardinali DP (1981) Melatonin. A mammalian pineal hormone. Endocr Rev. 2: 327-46
3. Gonzalez R. Sanches A, Ferguson J et al. (1991) Melatonin therapy of advanced human malignant melanoma. Melanoma research 1:237-243
4. Lesnikov VA, Pierpaoli W (1994) Pineal cross-transplantation (old-toyoung and vice versa) as evidence for an endogeneous "aging clock". Ann N Y Acad Sci 31:56-60
5. Allain D, Ravault JP, Panaretto BA, Rougeot J (1986) Effects of pinealectomy on photoperiodic control of hair follicle activity in the Limousine ram: possible relationship with plasma prolactin levels. J Pin Res 3: 25-32
6. Lincoln GA, Klansdorf H, Anderson N (1980) Photoperiodic control of thyroid function and wool and horn growth in rams and the effect of cranial sympathectomy. Endocrinology 107:1543-1548
7. Ryder ML. Cashmere, Mohair and Other Luxury Animal Fibres for the Breeder and Spinner. Itchen, Southhampton, 1987, pp 1-23
8. Webster JR, Barrell GK (1985) Advancement of reproductive activity, seasonal reduction in prolactin secretion and seasonal pelage changes in pubertal red deer hinds (Cervus elaphus) subjected to artificially shortened daily photoperiod or daily melatonin treatments. J Reprod Fertil 73:255-260
9. Bissonette TH, Wilson E (1939) Shortening daylight periods between May 15 and September 12 and the pelt cycle in mink. Science 89:418-419
10. Welch RAS, Grunsey M, Betteridge K, Mitchell RJ (1990) Goat fibre response to melatonin given in spring in two consecutive years. Proc. NZ Soc Anim Prod 50:335-338
11. Nixon AJ, Choj VJ, Parry AL, Pearson AJ (1993) Fiber growth initiation in hair follicles of goats treated with melatonin, J Exp Zool 267:47-56
12. Ibraheem M, Galbraith H, Scaife J, Ewen S (1994) Growth of secondary hair follicles of the Cashmere goat in vitro and their response to prolactin and melatonin. J Anat 185:135-142
13. Ebling FJ, Rook A: Hair. In: Rook A, Wilkinson DS, Ebling FJ (eds.): Textbook of Dermatology, Blackwell, Oxford, 1972, pp 1355-1425
14. Hamilton JB, (1942) Male hormone stimulation is prerequisite and an incitant in common baldness. Am J Anat 71:451-480
15. Ludwig E (1977) Classificatian of the types of androgenetic alopecia (common baldness) occurring in the female sex. Brit J Derm 97 237-254
16. Orfanos CE, Haar und Haarkrankheiten, Gustav Fischer Verlag, Stuttgart-New York, 1991, S. 573-584
17. Kauffmann KD, Olsen EA, Whiting D et al.(1998), Finasteride in the treatment of men with androgenetic alopecia. J Am Acad Dermatol, (1998) 39:578-589

## Patentansprüche

1. Verwendung einer Zusammensetzung, bestehend aus Melatonin, geeigneten Träger-, Verdünnungs- und Hilfsstoffen und Vitaminen, zur Herstellung eines Mittels zur Behandlung der androgenetischen und diffusen Alopezie vom weiblichen Typ.

2. Verwendung nach Anspruch 1,
**dadurch gekennzeichnet,**
**dass** das Mittel topisch applizierbar ist.

3. Verwendung nach Anspruch 1 oder 2,
**dadurch gekennzeichnet,**
**dass** das Mittel als Spray, Lösung, Lotion, Creme oder Salbe apptizierbar ist.

4. Verwendung nach einem der vorhergehenden Ansprüche,
**dadurch gekennzeichnet,**
**dass** das Melatonin-Präparat eine Wirkstoff-Konzentration von 0,001 bis 1 % (Gew./Gew.) aufweist.

5. Verwendung nach einem der vorhergehenden Ansprüche,
**dadurch gekennzeichnet,**
**dass** die täglich applizierte Menge des Mittels eine Wirkstoffdosis von 0,01 bis 10 mg pro Tag enthält.

6. Verwendung nach einem der vorhergehenden Ansprüche,
**dadurch gekennzeichnet,**
**dass** eine Reduktion der Telogenrate erzielt wird.

7. Verwendung nach einem der vorhergehenden Ansprüche,
**dadurch gekennzeichnet,**
**dass** eine Zunahme der Anagenrate erzielt wird.

8. Verwendung nach einem der vorhergehenden Ansprüche,
**dadurch gekennzeichnet,**
**dass** eine Vergrößerung des Haardurchmessers oder/und eine Erhöhung der Zerreißkraft der Haare erzielt wird.

9. Verwendung einer Zusammensetzung, umfassend Melatonin, geeigneten Träger-, Verdünnungs- und Hilfsstoffen und Vitaminen, zur Herstellung eines Mittels zur Vergrößerung des Haardurchmessers oder/und Erhöhung der Dehnungsfähigkeit bei diffuser Alopezie vom weiblichen Typ.

10. Verwendung nach Anspruch 9 bei okzipitalen Haaren.

11. Verwendung nach Anspruch 9 oder 10 bei weiblichen Patientinnen.

12. Verwendung von Melatonin zur Herstellung eines Mittels zur Behandlung der androgenetischen und diffusen Alopezie vom weiblichen Typ durch topisches Aufbringen auf die Kopfhaut ohne Vorbehandlung.

## Claims

1. Use of a composition consisting of melatonin, suitable vehicles, diluents and adjuvants and vitamins to produce an agent for treating androgenetic and diffuse alopecia of the female type.

2. Use as claimed in claim 1,
**characterized in that**
the agent can be applied topically.

3. Use as claimed in claim 1 or 2,
**characterized in that**
the agent can be applied as a spray, solution, lotion, cream or ointment.

4. Use as claimed in one of the previous claims,
**characterized in that**
the melatonin preparation has an active substance concentration of 0.001 to 1 % (weight/weight).

5. Use as claimed in one of the previous claims,
**characterized in that**
the amount of the agent that is applied daily contains an active substance dose of 0.01 to 10 mg per day.

6. Use as claimed in one of the previous claims,
**characterized in that**
a reduction in the telegenic rate is achieved.

7. Use as claimed in one of the previous claims,
**characterized in that**
an increase in the anagenic rate is achieved.

8. Use as claimed in one of the previous claims,
**characterized in that**
an enlargement of the hair diameter or/and an increase in the tearing strength of the hairs is achieved.

9. Use of a composition comprising melatonin, suitable vehicles, diluents and adjuvants and vitamins to produce an agent to enlarge the hair diameter or/and to increase the extensibility in diffuse alopecia of the female type.

10. Use as claimed in claim 9 for occipital hairs.

11. Use as claimed in claim 9 or 10 in female patients.

12. Use of melatonin to produce an agent for treating androgenetic and diffuse alopecia of the female type by topical application on the scalp without pretreatment.

## Revendications

1. Utilisation d'une composition consistant en mélatonine, en supports, diluants et adjuvants appropriés et en vitamines, pour la préparation d'un agent pour le traitement de l'alopécie androgénétique et diffuse de type féminin.

2. Utilisation selon la revendication 1 **caractérisée en ce que** l'agent est applicable par voie topique.

3. Utilisation selon la revendication 1 ou 2 **caractérisée en ce que** l'agent est applicable sous forme de spray, de solution, de lotion, de crème ou de pommade.

4. Utilisation selon l'une des revendications précédentes **caractérisée en ce que** la préparation de mélatonine présente une concentration de principe actif de 0,001 à 1 % (masse/masse).

5. Utilisation selon l'une des revendications précédentes **caractérisée en ce que** la quantité d'agent appliquée quotidiennement contient une dose de principe actif de 0,01 à 10 mg par jour.

6. Utilisation selon l'une des revendications précédentes **caractérisée en ce qu'**une réduction du taux de télogènes est obtenue.

7. Utilisation selon l'une des revendications précédentes **caractérisée en ce qu'**une augmentation du taux d'anagènes est obtenue.

8. Utilisation selon l'une des revendications précédentes **caractérisée en ce qu'**une augmentation du diamètre des cheveux et/ou une élévation de la force de rupture des cheveux sont obtenues.

9. Utilisation d'une composition comprenant de la mélatonine, des supports, diluants et adjuvants appropriés et des vitamines, pour la préparation d'un agent pour l'augmentation du diamètre des cheveux et/ou l'élévation de la capacité d'allongement dans le cas d'alopécie diffuse de type féminin.

10. Utilisation selon la revendication 9 dans le cas de cheveux occipitaux.

11. Utilisation selon la revendication 9 ou 10 dans le cas de patientes féminines.

12. Utilisation de la mélatonine pour la préparation d'un agent pour le traitement de l'alopécie androgénétique et diffuse de type féminin par application topique sur le cuir chevelu sans prétraitement.
